# EUROPÄISCHE PATENTANMELDUNG

(11) **EP 2 179 993 A1**
(43) Veröffentlichungstag der Anmeldung: **28.04.2010**
(21) Anmeldenummer: 08167117.4
(22) Anmeldetag: 21.10.2008
(51) Int. Cl.: C07D 239/47, A61K 31/505, A61P 35/00

(54) **Sulfoxidsubstituierte Anilinopyrimidinderivative als CDK-Inhibitoren, deren Herstellung und Verwendung als Arzneimittel**

(71) Anmelder: Bayer Schering Pharma Aktiengesellschaft, 13353 Berlin (DE)
(72) Erfinder: Lücking, Ulrich, 10317 Berlin (DE); Siemeister, Gerhard, 13503 Berlin (DE); Lienau, Philip, 10997 Berlin (DE); Lautelat, Rolf, 16548 Glienicke/Nordbahn (DE); Schulze, Julia, 10407 Berlin (DE)

(57) **Zusammenfassung**

Die Erfindung betrifft sulfoxidsubsituierte Anilino-Pyrimidinderivate der Formel (I), 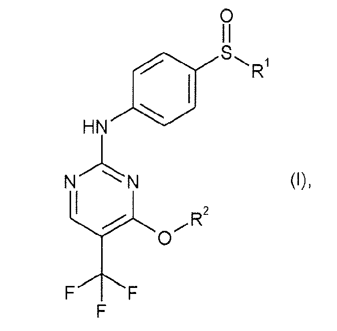 deren Verfahren zur Herstellung, sowie deren Verwendung als Medikament zur Behandlung verschiedener Erkrankungen.

## Beschreibung

Die vorliegende Erfindung betrifft sulfoxidsubsituierte Anilino-Pyrimidinderivate, deren Verfahren zur Herstellung, sowie deren Verwendung als Medikament zur Behandlung verschiedener Erkrankungen.

Die Zyklin-abhängigen Kinasen (cyclin-dependent kinase, CDK) sind eine Enzymfamilie, die eine wichtige Rolle bei der Regulation des Zellzyklusses spielt und somit ein besonders interessantes Ziel für die Entwicklung kleiner inhibitorischer Moleküle darstellt. Selektive Inhibitoren der CDKs können zur Behandlung von Krebs oder anderen Erkrankungen, die Störungen der Zellproliferation zur Ursache haben, verwendet werden.

Pyrimidine und Analoga sind bereits als Wirkstoffe beschrieben wie beispielsweise die 2-Anilino-Pyrimidine als Fungizide (DE 4029650) oder substituierte Pyrimidinderivate zur Behandlung von neurologischen oder neurodegenerativen Erkrankungen (WO 99/19305). Als CDK-Inhibitoren werden unterschiedlichste Pyrimidinderivate beschrieben, beispielsweise 2-Amino-4-substituierte Pyrimidine (WO 01/ 14375), Purine (WO 99/02162), 5-Cyano-Pyrimidine (WO 02/04429), Anilinopyrimidine (WO 00/12486) und 2-Hydroxy-3-N,N-dimethylaminopropoxy-Pyrimidine (WO 00/39101).

Insbesondere wurden in WO 02/096888 und WO 03/076437 Pyrimidinderivate offenbart, die inhibitorische Wirkungen bezüglich CDKs aufweisen.

WO 2005/037800 offenbart offene sulfoximinsubsituierte Anilino-Pyrimidinderivate als Inhibitoren der Zyklin-abhängigen Kinasen. Beispielhaft belegt sind Strukturen, die in der 5-Position des Pyrimidins entweder nicht oder mit Halogen, insbesondere mit Brom substituiert sind. Einen 5-Trifluormethylsubstituenten weist keine der spezifisch offenbarten Strukturen auf.

WO 2003/032997 offenbart sulfonsubstituierte Anilinopyrimidine, für die allerdings in der Position 4 des Pyrimidins obligat ein stickstoffhaltiger Rest vorgesehen ist.

Ausgehend von diesem Stand der Technik besteht die Aufgabe der vorliegenden Erfindung Verbindungen bereitzustellen, die die Aktivität der Zyklin-abhängigen Kinasen stärker inhibieren als die Verbindungen des Standes der Technik. Weiterhin sollen die Verbindungen selektiver gegenüber der Inhibition der VEGF-Rezeptorkinase-2 (VEGF-R2) sein. Die Verbindungen sollen gut permeabel in absorptiver Richtung und gering permeabel in Efflux-Richtung sein. Insbesondere sollen die Verbindungen auch in Chemotherapie-resistenten Tumorzellen stark antiproliferativ wirken.

Es wurde nun gefunden, dass Verbindungen der allgemeinen Formel (1) 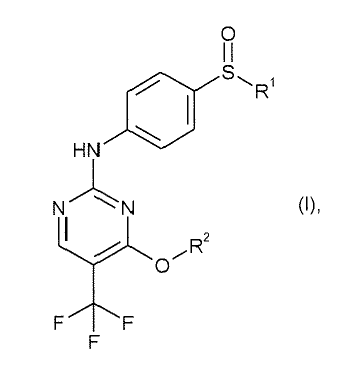 in der
- R¹: für einen C₁-C₆-Alkyl-, C₂-C₆-Alkenyl-, C₂-C₆-Alkinylrest oder einen C₃-C₇-Cycloalkyl oder Phenylring steht, jeweils gegebenenfalls mit Hydroxy, -NR³R⁴, Cyano, Halogen, -CF₃, C₁-C₆-Alkoxy, -OCF₃ und/oder C₁-C₆-Alkyl ein- oder mehrfach, gleich oder verschieden substituiert, und
- R²: für einen C₁-C₁₀-Alkyl-, C₃-C₁₀-Alkenyl- oder C₃-C₁₀-Alkinylrest oder einen C₃-C₇-Cycloalkylring steht, jeweils gegebenenfalls ein- oder mehrfach, gleich oder verschieden substituiert mit a) Halogen, Hydroxy, -NR³R⁴, Cyano, -CF₃, -OCF₃, und/oder b) C₁-C₆-Alkoxy, C₁-C₆-Alkyl, C₂-C₆-Alkenyl, C₂-C₆-Alkinyl, oder C₃- C₈-Cycloalkyl, -O-CH₂-Phenyl, Cₙ-Alkoxycarbonyl, jeweils gegebenenfalls selbst ein- oder mehrfach, gleich oder verschieden mit Halogen, Hydroxy, einem C₁-C₆-Alkyl, C₁-C₆- Alkoxy, -NR³R⁴, -CF₃ und/oder-OCF₃ substituiert, und
- R³ und R⁴: unabhängig voneinander für Wasserstoff und/oder einen C₁-C₆- Alkylrest, C₂-C₆-Alkenylrest, C₃-C₈-Cycloalkyl- und/oder Phenylring, einen Heterocyclylring mit 3 bis 8 Ringatomen und/oder einen monocyclischen Heteroarylring, gegebenenfalls mit Hydroxy, -NR⁵R⁶, Cyano, Halogen, -CF₃, C₁-C₆-Alkoxy und/oder -OCF₃ ein- oder mehrfach, gleich oder verschieden substituiert sind, oder
- R³ und R⁴: gemeinsam mit dem Stickstoffatom einen 5- bis 7-gliedrigen Ring bilden, der gegebenenfalls zusätzlich zum Stickstoffatom 1 oder 2 weitere Heteroatome enthält und der mit Hydroxy, -NR⁵R⁶ Cyano, Halogen, -CF₃, C₁-C₆-Alkoxy und/oder -OCF₃ ein- oder mehrfach, gleich oder verschieden substituiert sein kann, und
- R⁵ und R⁶: unabhängig voneinander für Wasserstoff oder einen C₁-C₆-Alkylrest stehen, der gegebenenfalls mit Hydroxy, Cyano, Halogen, -CF₃, C₁-C₆-Alkoxy und/oder -OCF₃ ein- oder mehrfach, gleich oder verschieden substituiert ist,
sowie deren Salze, Diatereomere und Enantiomere.

Der Erfindung liegen folgende Definitionen zu Grunde:

### Cn-Alkyl:

Monovalenter, geradkettiger oder verzweigter, gesättigter Kohlenwasserstoffrest mit n Kohlenstoffatomen.

Ein C₁-C₆ Alkylrest umfasst unter anderem beispielsweise:
Methyl-, Ethyl-, Propyl-, Butyl-, Pentyl-,Hexyl-, *iso*-Propyl-, *iso*-Butyl-, *sec*-Butyl, *tert-*Butyl-, *iso*-Pentyl-, 2-Methylbutyl-, 1-Methylbutyl-, 1-Ethylpropyl-, 1,2-Dimethylpropyl, *n*eo-Pentyl-, 1,1-Dimethylpropyl-, 4-Methylpentyl-, 3-Methylpentyl-, 2-Methylpentyl-, 1-Methylpentyl-, 2-Ethylbutyl-, 1-Ethylbutyl-, 3,3-Dimethylbutyl-, 2,2-Dimethylbutyl-, 1,1-Dimethylbutyl-, 2,3-Dimethylbutyl-, 1,3-Dimethylbutyl- 1,2-Dimethylbutyl-.

Bevorzugt ist ein Methyl-, Ethyl-, Propyl- oder Isopropylrest.

### Cₙ-Alkenyl:

monovalenter, geradkettiger oder verzweigter Kohlenwasserstoffrest mit n Kohlenstoffatomen und mindestens einer Doppelbindung.

Ein C₂-C₁₀ Alkenylrest umfasst unter anderem beispielsweise:
Vinyl-, Allyl-, (E)-2-Methylvinyl-, (Z)-2-Methylvinyl-, Homoallyl-, (E)-But-2-enyl-, (Z)-But-2-enyl-, (E)-But-1-enyl-, (Z)-But-1-enyl-, Pent-4-enyl-, (E)-Pent-3-enyl-, (Z)-Pent-3-enyl-, (E)-Pent-2-enyl-, (Z)-Pent-2-enyl-, (E)-Pent-1-enyl-, (Z)-Pent-1-enyl, Hex-5-enyl-, (E)-Hex-4-enyl-, (Z)-Hex-4-enyl-, (E)-Hex-3-enyl-, (Z)-Hex-3-enyl-, (E)-Hex-2-enyl-, (Z)-Hex-2-enyl-, (E)-Hex-1-enyl-, (Z)-Hex-1-enyl-, Isopropenyl-, 2-Methylprop-2-enyl-, 1-Methylprop-2-enyl-, 2-Methylprop-1-enyl-, (E)-1-Methylprop-1-enyl-, (Z)-1-Methylprop-1-enyl-, 3-Methylbut-3-enyl-, 2-Methylbut-3-enyl-, 1-Methylbut-3-enyl-, 3-Methylbut-2-enyl-, (E)-2-Methylbut-2-enyl-, (Z)-2-Methylbut-2-enyl-, (E)-1-Methylbut-2-enyl-, (Z)-1-Methylbut-2-enyl-, (E)-3-Methylbut-1-enyl-, (Z)-3-Methylbut-1-enyl-, (E)-2-Methylbut-1-enyl-, (Z)-2-Methylbut-1-enyl-, (E)-1-Methylbut-1-enyl-, (Z)-1-Methylbut-1-enyl-, 1,1-Dimethylprop-2-enyl-, 1-Ethylprop-1-enyl-, 1-Propylvinyl-, 1-Isopropylvinyl-, 4-Methylpent-4-enyl-, 3-Methylpent-4-enyl-, 2-Methylpent-4-enyl-, 1-Methylpent-4-enyl-, 4-Methylpent-3-enyl-, (E)-3-Methylpent-3-enyl-, (Z)-3-Methylpent-3-enyl-, (E)-2-Methylpent-3-enyl-, (Z)-2-Methylpent-3-enyl-, (E)-1-Methylpent-3-enyl-, (Z)-1-Methylpent-3-enyl-, (E)-4-Methylpent-2-enyl-, (Z)-4-Methylpent-2-enyl-, (E)-3-Methylpent-2-enyl-, (Z)-3-Methylpent-2-enyl-, (E)-2-Methylpent-2-enyl-, (Z)-2-Methylpent-2-enyl-, (E)-1-Methylpent-2-enyl-, (Z)-1-Methylpent-2-enyl-, (E)-4-Methylpent-1-enyl-, (Z)-4-Methylpent-1-enyl-, (E)-3-Methylpent-1-enyl-, (Z)-3-Methylpent-1-enyl-, (E)-2-Methylpent-1-enyl-, (Z)-2-Methylpent-1-enyl-, (E)-1-Methylpent-1-enyl-, (Z)-1-Methylpent-1-enyl-, 3-Ethylbut-3-enyl-, 2-Ethylbut-3-enyl-, 1-Ethylbut-3-enyl-, (E)-3-Ethylbut-2-enyl-, (Z)-3-Ethylbut-2-enyl-, (E)-2-Ethylbut-2-enyl-, (Z)-2-Ethylbut-2-enyl-, (E)-1-Ethylbut-2-enyl-, (Z)-1-Ethylbut-2-enyl-, (E)-3-Ethylbut-1-enyl-, (Z)-3-Ethylbut-1-enyl-, 2-Ethylbut-1-enyl-, (E)-1-Ethylbut-1-enyl-, (Z)-1-Ethylbut-1-enyl-, 2-Propylprop-2-enyl-, 1-Propylprop-2-enyl-, 2-Isopropylprop-2-enyl-, 1-Isopropylprop-2-enyl-, (E)-2-Propylprop-1-enyl-, (Z)-2-Propylprop-1-enyl-, (E)-1-Propylprop-1-enyl-, (Z)-1-Propylprop-1-enyl-, (E)-2-Isopropylprop-1-enyl-, (Z)-2-Isopropylprop-1-enyl-, (E)-1-Isopropylprop-1-enyl-, (Z)-1-Isopropylprop-1-enyl-, (E)-3,3-Dimethylprop-1-enyl-, (Z)-3,3-Dimethylprop-1-enyl-, 1-(1,1-Dimethylethyl)ethenyl.

Bevorzugt ist ein Vinyl- oder Allylrest.

### Cₙ-Alkinyl:

Monovalenter, geradkettiger oder verzweigter Kohlenwasserstoffrest mit n Kohlenstoffatomen und mindestens einer Dreifachbindung.

Ein C₂-C₁₀ Alkinylrest umfasst unter anderem beispielsweise:
Ethinyl-, Prop-1-inyl-, Prop-2-inyl-, But-1-inyl-, But-2-inyl-, But-3-inyl-, Pent-1-inyl-, Pent-2-inyl-, Pent-3-inyl-, Pent-4-inyl-, Hex-1-inyl-, Hex-2-inyl-, Hex-3-inyl-, Hex-4-inyl-, Hex-5-inyl-, 1-Methylprop-2-inyl-, 2-Methylbut-3-inyl-, 1-Methylbut-3-inyl-, 1-Methylbut-2-inyl-, 3-Methylbut-1-inyl-, 1-Ethylprop-2-inyl-, 3-Methylpent-4-inyl, 2-Methylpent-4-inyl, 1-Methylpent-4-inyl, 2-Methylpent-3-inyl, 1-Methylpent-3-inyl, 4-Methylpent-2-inyl, 1-Methylpent-2-inyl, 4-Methylpent-1-inyl, 3-Methylpent-1-inyl, 2-Ethylbut-3-inyl-, 1-Ethylbut-3-inyl-, 1-Ethylbut-2-inyl-, 1-Propylprop-2-inyl-, 1-Isopropylprop-2-inyl-, 2,2-Dimethylbut-3-inyl-, 1,1-Dimethylbut-3-inyl-, 1,1-Dimethylbut-2-inyl- oder 3,3-Dimethylbut-1-inyl-.

Bevorzugt ist ein Ethinyl-, Prop-1-inyl- oder Prop-2-inyl-rest.

### Cₙ-Cycloalkyl:

Monovalenter, cyclischer Kohlenwasserstoffring mit n Kohlenstoffatomen.

### C₃-C₇-Cyclolalkylring umfasst:

Cyclopropyl, Cyclobutyl, Cyclopentyl, Cyclohexyl- und Cycloheptyl.

Bevorzugt ist ein Cyclopropyl-, Cylopentyl- oder ein Cyclohexylring.

### Cₙ-Alkoxy:

Geradkettiger oder verzweigter Cₙ-Alkyletherrest der Formel -OR mit R= Cₙ-Alkyl.

### Cₙ-Alkoxycarbonyl

Cₙ-Alkoxycarbonyl steht für die Gruppe -C(O)-O-Cₙ-Alkyl.

In der Regel ist p gleich 1 bis 6, bevorzugt 1 bis 4, und besonders bevorzugt 1 bis 3. Beispielhaft und vorzugsweise seien genannt:
Methoxycarbonyl, Ethoxycarbonyl, Propoxycarbonyl, Isopropoxycarbonyl, tert.-Butoxycarbonyl, n-Pentoxycarbonyl und n-Hexoxycarbonyl.

### Halogen

Die Bezeichnung Halogen umfasst Fluor, Chlor, Brom und Jod.

Bevorzugt ist Fluor.

In der allgemeinen Formel (1) kann R¹ stehen für:
einen C₁-C₆-Alkyl-, C₂-C₆-Alkenyl-, C₂-C₆-Alkinylrest oder einen C₃-C₇-Cycloalkyl oder Phenylring, jeweils gegebenenfalls mit Hydroxy, -NR³R⁴, Cyano, Halogen, -CF₃, C₁-C₆-Alkoxy, -OCF₃ und/oder C₁-C₆-Alkyl ein- oder mehrfach, gleich oder verschieden substituiert.

Bevorzugt steht R¹ für einen C₁-C₆-Alkyl-, C₂-C₆-Alkenyl-, C₂-C₆-Alkinylrest oder einen C₃-C₇-Cydoalkyl oder Phenylring, jeweils gegebenenfalls mit Hydroxy, Cyano, Halogen, -CF₃, C₁-C₆-Alkoxy, -OCF₃ und/oder C₁-C₆-Alkyl ein- oder mehrfach, gleich oder verschieden substituiert.

Mehr bevorzugt steht R¹ für einen C₁-C₆-Alkyl- oder C₂-C₆-Alkenylrest oder einen C₃-C₇-Cycloalkyl- oder Phenylring, jeweils gegebenenfalls mit Hydroxy, Cyano, Halogen und/oder C₁-C₆-Alkyl ein- oder mehrfach, gleich oder verschieden substituiert.

Besonders bevorzugt steht R¹ für eine C₁-C₆-Alkylgruppe oder einen C₃-C₇-Cycloalkylring.

Außerordentlich bevorzugt steht R¹für eine Methylgruppe.

In der allgemeinen Formel (I) kann R² stehen für:
einen C₁-C₁₀-Alkyl-, C₃-C₁₀-Alkenyl- oder C₃-C₁₀-Alkinylrest oder einen C₃-C₇-Cycloalkylring, jeweils gegebenenfalls ein- oder mehrfach, gleich oder verschieden substituiert mit
   a) Halogen, Hydroxy, -NR³R⁴, Cyano, -CF₃, -OCF₃, und/oder
   b) C₁-C₆-Alkoxy, C₁-C₆-Alkyl, C₂-C₆-Alkenyl, C₂-C₆-Alkinyl, oder C₃-C₈-Cycloalkyl, -O-CH₂-Phenyl, Cₙ-Alkoxycarbonyl, jeweils gegebenenfalls selbst ein- oder mehrfach, gleich oder verschieden mit Halogen, Hydroxy, einem C₁-C₆-Alkyl, C₁-C₆-Alkoxy, -NR³R⁴, -CF₃ und/oder -OCF₃ substituiert.

Bevorzugt steht R² für einen C₁-C₁₀-Alkyl-, C₃-C₁₀-Alkenyl- oder C₃-C₁₀-Alkinylrest oder einen C₃-C₇-Cycloalkylring, jeweils gegebenenfalls ein- oder mehrfach, gleich oder verschieden substituiert mit Halogen, Hydroxy, Cyano, -CF₃, -OCF₃, und/oder C₁-C₆-Alkoxy, C₁-C₆-Alkyl, jeweils gegebenenfalls selbst ein- oder mehrfach, gleich oder verschieden substituiert mit Halogen oder Hydroxy.

Mehr bevorzugt steht R² für einen C₂-C₆-Alkyl-, C₃-C₆-Alkenyl- oder C₃-C₆-Alkinylrest oder einen C₃-C₇-Cycloalkylring, der gegebenenfalls ein- oder mehrfach substituiert ist mit Hydroxy, Halogen, -CF₃ und/oder C₁-C₃-Alkoxy.

Besonders bevorzugt steht R² für die Gruppe mit der Teilformel (I-_{R}²), 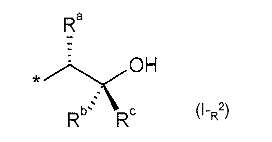 in der
- R^{a}: für eine Methyl-, Ethyl-, Propyl- oder Isopropylgruppe steht, und
- R^{b} und R^{c}: unabhängig voneinander für Wasserstoff, eine Methyl- oder Ethylgruppe stehen.

Formel (la) fasst diese Gruppe von Verbindungen zusammen.

Bevorzugt stehen R^{a} und R^{b} für eine Methylgruppe und R^{c} für Wasserstoff oder eine Methylgruppe.

In der allgemeinen Formel (1) können R³ und R⁴ unabhängig voneinander stehen für: Wasserstoff und/oder einen C₁-C₆-Alkylrest, C₂-C₆-Alkenylrest, C₃-C₈-Cycloalkyl-und/oder Phenylring, einen Heterocyclylring mit 3 bis 8 Ringatomen und/oder einen monocyclischen Heteroarylring, gegebenenfalls mit Hydroxy, -NR⁵R⁶, Cyano, Halogen, -CF₃, C₁-C₆-Alkoxy und/oder -OCF₃ ein- oder mehrfach, gleich oder verschieden substituiert sind,
oder
R³ und R⁴ bilden gemeinsam mit dem Stickstoffatom einen 5- bis 7-gliedrigen Ring, der gegebenenfalls zusätzlich zum Stickstoffatom 1 oder 2 weitere Heteroatome enthält und der mit Hydroxy, -NR⁵R⁶, Cyano, Halogen, -CF₃, C₁-C₆-Alkoxy und/oder -OCF₃ ein- oder mehrfach, gleich oder verschieden substituiert sein kann.

Bevorzugt stehen R³ und R⁴ unabhängig voneinander für Wasserstoff und/oder einen C₁-C₆-Alkylrest, C₃-C₈-Cycloalkyl- und/oder Phenylring, gegebenenfalls mit Hydroxy, Cyano, Halogen, -CF₃, C₁-C₆-Alkoxy und/oder -OCF₃ ein- oder mehrfach, gleich oder verschieden substituiert.

In der allgemeinen Formel (1) können R⁵ und R⁶ unabhängig voneinander stehen für: Wasserstoff oder einen C₁-C₆-Alkylrest stehen, der gegebenenfalls mit Hydroxy, Cyano, Halogen, -CF₃, C₁-C₆-Alkoxy und/oder -OCF₃ ein- oder mehrfach, gleich oder verschieden substituiert ist.

Bevorzugt stehen R⁵ und R⁶ unabhängig voneinanderfür Wasserstoff und/oder einen C₁-C₃-Alkylrest.

Eine bevorzugte Untergruppe bilden Verbindungen der allgemeinen Formel (1), in der
- R¹: für einen C₁-C₆-Alkyl-, C₂-C₆-Alkenyl-, C₂-C₆-Alkinylrest oder einen C₃-C₇-Cycloalkyl oder Phenylring steht, jeweils gegebenenfalls mit Hydroxy, Cyano, Halogen, -CF₃, C₁-C₆-Alkoxy, -OCF₃ und/oder C₁-C₆-Alkyl ein- oder mehrfach, gleich oder verschieden substituiert, und
- R²: für einen C₁-C₁₀-Alkyl-, C₃-C₁₀-Alkenyl- oder C₃-C₁₀-Alkinylrest oder einen C₃-C₇-Cycloalkylring steht, jeweils gegebenenfalls ein- oder mehrfach, gleich oder verschieden substituiert mit Halogen, Hydroxy, Cyano, -CF₃, -OCF₃, und/oder C₁-C₆-Alkoxy, C₁-C₆-Alkyl, jeweils gegebenenfalls selbst ein- oder mehrfach, gleich oder verschieden substituiert mit Halogen oder Hydroxy,
sowie deren Salze, Diatereomere und Enantiomere.

Eine mehr bevorzugte Untergruppe bilden Verbindungen der allgemeinen Formel (I) in der
- R¹: für einen C₁-C₆-Alkyl- oder C₂-C₆-Alkenylrest oder einen C₃-C₇- Cycloalkyl- oder Phenylring steht, jeweils gegebenenfalls mit Hydroxy, Cyano, Halogen und/oder C₁-C₆-Alkyl ein- oder mehrfach, gleich oder verschieden substituiert, und
- R²: für einen C₂-C₆-Alkyl-, C₃-C₆-Alkenyl- oder C₃-C₆-Alkinylrest oder einen C₃-C₇-Cycloalkylring steht, der gegebenenfalls ein- oder mehrfach substituiert ist mit Hydroxy, Halogen, -CF₃ und/oder C₁-C₃- Alkoxy,
sowie deren Salze, Diatereomere und Enantiomere.

Eine besonders bevorzugte Untergruppe bilden Verbindungen der allgemeinen Formel (la) 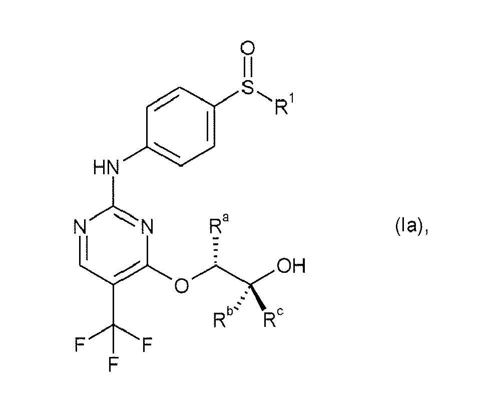 in der
- R¹: für eine C₁-C₆-Alkylgruppe oder einen C₃-C₇-Cycloalkylring steht, und
- R^{a}: für eine Methyl-, Ethyl-, Propyl- oder Isopropylgruppe steht, und
- R^{b} und R^{c}: unabhängig voneinander für Wasserstoff, eine Methyl- oder Ethylgruppe stehen,
sowie deren Salze, Diatereomere und Enantiomere.

Eine bevorzugte Untergruppe der Verbindungen der allgemeinen Formel (la) bildet die Gruppe von Verbindungen,
in der
- R¹: für eine Methylgruppe steht, und
- R^{a} und R^{b}: für eine Methylgruppe stehen, und
- R^{c}: für Wasserstoff oder eine Methylgruppe steht,
sowie deren Salze, Diatereomere und Enantiomere.

Die erfindungsgemäßen Verbindungen eignen sich zur Behandlung
- von Krebs, wie solide Tumore, Tumormetastasen, und Hämatologische Tumoren, insbesondere:
   Kopf- und Halstumore; Lungen- und Bronchialtumore; Gastrointestinaltumore wie z.B. Magenkarzinom, Kolorektales Karzinom, Pankreaskarzinom, Hepatozelluläres Karzinom; Endokin aktive Tumore; Mammakarzinome und
   Gynäkologische Tumore; Urogenitaltumore, wie z.B. Nierenzellkarzinom, Harnblasenkarzinom, Prostatakarzinom; Tumore der Haut; Sarkome; Leukämien und Lymphome.
- von viralen Erkrankungen, sowie
- von kardiovaskulären Erkrankungen wie Stenosen, Arteriosklerosen und Restenosen, Stent-induzierte Restenosen.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen. Dabei ist auf Remington's Pharmaceutical Science, 15th ed. Mack Publishing Company, East Pennsylvania (1980) hinzuweisen.

Die pharmazeutischen Formulierungen können
in fester Form, zum Beispiel als Tabletten, Dragees, Pillen, Suppositorien, Kapseln, transdermale Systeme oder
in halbfester Form , zum Beispiel als Salben, Cremes, Gele, Suppositiorien, Emulsionen oder
in flüssiger Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.
Für die parenterale Applikation kommen insbesondere Suspensionen, Emulsionen und vor allem Lösungen in Frage.

### Herstellung der erfindungsgemäßen Verbindungen

Die nachfolgenden Beispiele erläutern die Herstellung der erfindungsgemäßen Verbindungen, ohne den Umfang der beanspruchten Verbindungen auf diese Beispiele zu beschränken.

Die erfindungsgemäßen Verbindungen können hergestellt werden durch ein Verfahren, das durch folgende Schritte gekennzeichnet ist:
a₁) Funktionalisierung der 4-Position von 2,4-Dichlor-5-jod-pyrimidin **(1)** durch Umsetzung mit einem Alkohol der Formel **(2)** unter Bildung eines Intermediates der Formel **(3)** 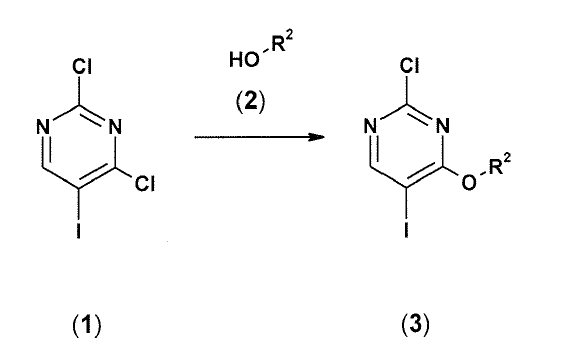 und nachfolgende Umsetzung von Intermediat (3) unter Bildung des 5-CF₃ Intermediates **(4)** 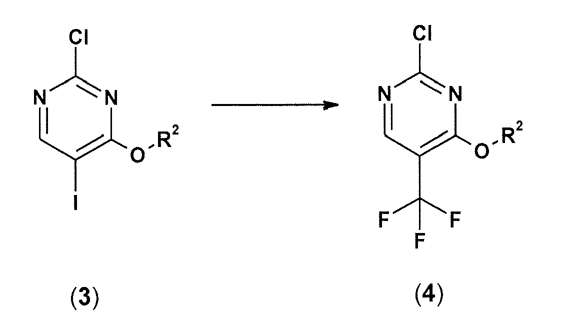 oder alternativ
a₂) direkte Umsetzung von 2,4-Dichlor-5-trifluormethyl-pyrimidin **(5)** und einem Alkohol der Formel **(2)** unter Bildung des 5-CF₃ Intermediates **(4).** 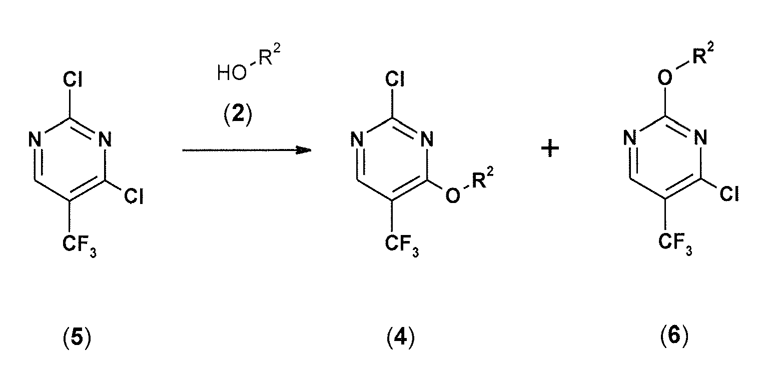
b₁) Oxidation eines Thioethers der Formel **(7)** zum Sulfoxid der Formel (8). 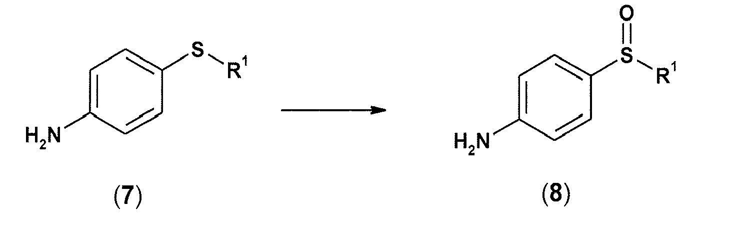 und anschließende Kupplung der Verbindungen der Formel **(4)** und **(8)** 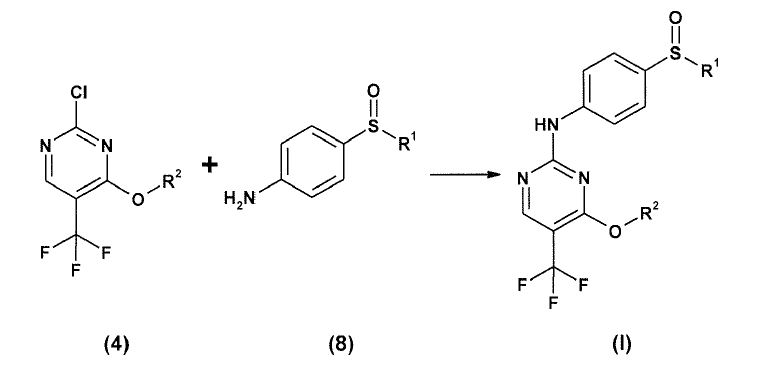 oder alternativ
b₂) Kupplung der Intermediate **(4)** und **(7)** unter Bildung des Produktes (9) erfolgen 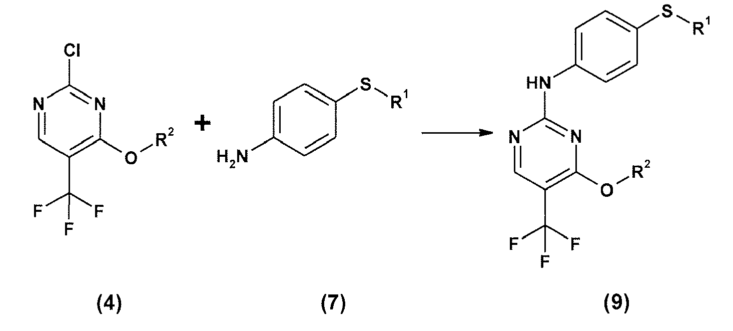 und anschließend Oxidation von **(9)** zum Sulfoxid 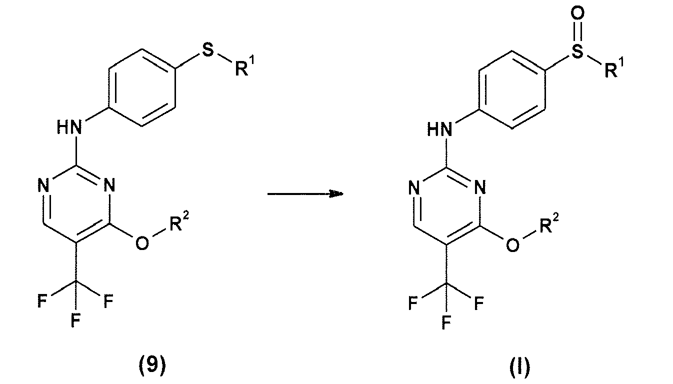 wobei die Substituenten R¹ und R² die oben in der allgemeinen Formel (1) angegebenen Bedeutungen aufweisen.

Gegebenfalls erfordert die Herstellung der erfindungsgemäßen Verbindungen das Einführen und anschließende Abspalten von Schutzgruppen (siehe z.B.: P.J. Kocienski, Protecting Groups, Georg Thieme Verlag Stuttgart, New York, 1994) wie z.B. in der Seitenkette der 4-Position.

### Verfahrensschritt a₁)

Die Umsetzung von 2,4-Dichlor-5-jod-pyrimidin **(1)** mit einem Alkohol der Formel **(2)** unter basischen Bedingungen ermöglicht die Darstellung eines Produktes der Formel **(3)** (siehe z.B.: (a) U. Lücking et al, WO 2007/071455). Besonders geeignet für die Darstellung ist die beschriebene Verwendung von Natriumhydrid.

Für den Austausch eines Halogens gegen eine Trifluormethylgruppe in einem stickstoffhaltigen Heteroaromaten stehen prinzipiell verschiedene Methoden zur Verfügung (siehe z.B: a) G.E. Carr, R.D. Chambers, T.F. Holmes, J. Chem. Soc. Perkin Trans. 1, 1988, 921; b) F. Cottet, M. Schlosser, Eur. J. Org. Chem. 2002, 327; c) F.G. Njoroge et al, J. Med. Chem 1997, 40, 4290).
Besonders geeignet für den Austausch des Jods in der 5-Position des Pyrimidins **(3)** gegen eine CF₃-Gruppe unter Bildung einer Verbindung der Formel **(4)** ist die beschriebene Verwendung Kupfer(1)jodid, Kaliumfluorid und (Trifluormethyl)-trimethylsilan in N-Methyl-2-pyrrolidinon und THF.

### Verfahrensschritt a₂)

Die Umsetzung von 2,4-Dichlor-5-trifluormethyl-pyrimidin **(5)** mit einem Alkohol der Formel **(2)** unter basischen Bedingungen ermöglicht die Darstellung der Produkte **(4)** und **(6).** Die Regioisomeren können in der Regel chromatographisch getrennt werden. (siehe z.B.: (a) T.M. Caldwell et al, WO 2006/081388, S. 50, Example 1, D). Besonders geeignet für die Darstellung ist die beschriebene Verwendung von Natriumhydrid.

### Verfahrensschritt b₁)

Eine Verbindung der Formel **(7)** wird zum Sulfoxid der Formel **(8)** oxidiert. Für die Überführung eines Thioethers in ein Sulfoxid stehen zahlreiche Methoden, auch stereoselektive, zur Verfügung (siehe z.B.: (a) M.H. Ali et al, Synthesis 1997, 764; b) M.C. Carreno, Chem. Rev. 1995, 95, 1717; c) I. Patel et al, Org. Proc. Res. Dev. 2002, 6, 225; c) N. Khiar et al, Chem. Rev. 2003, 103, 3651). Besonders geeignet ist die beschriebene Verwendung von Perjodsäure und Eisen(III)chlorid.

Eine Verbindung der Formel **(4)** kann anschließend mit einem Anilin der Formel **(8)** zu einer Verbindung der Formel **(I)** umgesetzt werden.

### Verfahrensschritt b₂)

Alternativ kann auch eine Verbindung der Formel **(4)** mit einem Anilin der Formel **(7)** zu einer Verbindung der Formel **(9)** umgesetzt werden.
Die anschließende Oxidation von **(9)** zum Sulfoxid liefert die gewünschten Verbindungen der Formel (I).

### Allgemeine Hinweise

Alle Reaktionen mit oxidations- oder hydrolyseempfindlichen Verbindungen wurden unter Argon und mit getrockneten Lösungsmitteln durchgeführt.
Die Substanzbenennung erfolgte unter Verwendung des Programms *Autonom 2000 Name,* welches in MDL ISIS Draw implementiert ist.

### Abkürzungen

| **Abkürzung** | **Bedeutung** |
|---|---|
| Ac | Acetyl |
| Aloc | Allyloxycarbonyl |
| Boc | tert-Butyloxycarbonyl |
| BOM | Benzyloxymethyl |
| br | Broad |
| Cl | Chemical ionisation |
| d | Dublett |
| dd | Dublett vom Dublett |
| DCM | Dichlormethan |
| DMF | *N,N*-Dimethylformamid |
| DMSO | Dimethylsulfoxid |
| ESI | Electrospray ionisation |
| HPLC | High performance liquid chromatography |
| m | Multiplett |
| MEM | (2-Methoxyethoxy)methyl |
| MOM | Methoxymethyl |
| MS | Mass spectrometry |
| MTM | Methylthiomethyl |
| NMP | N-Methyl-2-pyrrolidinon |
| NMR | Nuclear magnetic resonance spectroscopy: Chemische Verschiebung (δ) wird in ppm angegeben. |
| Pg | Protecting group umfassend Gruppen wie z.B. TMS, TES, TBDMS, TBDPS, TIPS, Benzyl, PMB, Trityl, Allyl, Aloc, MOM, MTM, MEM, BOM, SEM, THP. |
| PMB | p-Methoxybenzyl |
| q | Quartett |
| s | Singulett |
| SEM | β-(Trimethylsilyl)ethoxymethyl |
| TBDMS | Tert.-Butylsilyldimethyl |
| TBDPS | Tert.-Butylsilyldiphenyl |
| TEA | Triethylamin |
| TES | Triethylsilyl |
| THF | Tetrahydrofuran |
| THP | Tetrahydropyranyl |
| TIPS | Triisopropyl |
| TMS | Trimethylsilyl |
| tr | Triplett |

### Beispiel 1

### (2R,3R)-3-[2-(4-Methansulfinyl-phenylamino)-5-trifluormethyl-pyrimidin-4-yloxy]-butan-2-ol

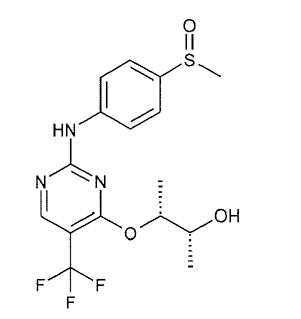

### 1a) Herstellung der Zwischenprodukte

### Verbindung 1.1

### (2R,3R)-3-Benzyloxy-butan-2-ol

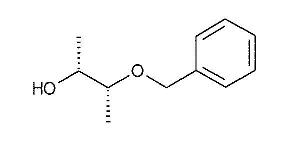

Eine Lösung von 4,0 g (44,4 mmol) (2R,3R)-Butan-2,3-diol in 300 ml THF wurde bei Raumtemperatur mit 5,0 g (44,6 mmol) Kalium-tert.-butylat versetzt und der Ansatz 15 Minuten refluxiert. Der Ansatz wurde auf ca. 50°C abgekühlt und mit 5,3 ml (44,6 mmol) Benzylbromid versetzt. Man refluxierte für 3 Stunden, danach wurde über Nacht bei Raumtemperatur gerührt. Der Ansatz wurde mit Essigester und Natriumchlorid-Lösung verdünnt und anschließend mit 1 N Chlorwasserstoff-Lösung (1 x) und Natriumchlorid-Lösung (2x) gewaschen. Die organische Phase wurde getrocknet (Na₂SO₄), filtriert und eingeengt. Der erhaltene Rückstand wurde chromatographisch (Hexan / Essigester 1:1) gereinigt. Man erhielt 3,4 g (18,9 mmol; Ausbeute: 43 %) des Produktes.
¹H-NMR (400 MHz, DMSO): δ = 7.35 (m, 4H), 7.28 (m, 1H), 4.52 (m, 3H), 3.67 (m, 1H), 3.37 (m, 1H), 1.05 (d, 3H), 1.01 (d, 3H).

### Verbindung 1.2

### 4-((1R,2R)-2-Benzyloxy-1-methyl-propoxy)-2-chlor-5-jod-pyrimidin

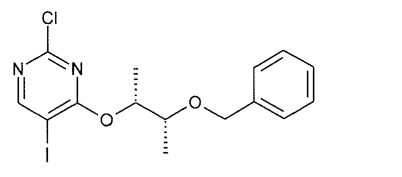

8,55 g (47,4 mmol) (2R,3R)-3-Benzyloxy-butan-2-ol in 56 ml Diethylether wurden bei 0°C unter Rühren portionsweise mit 2,07 g Natriumhydrid (55%) versetzt. Nach 10 Minuten wurde das Eisbad entfernt und weitere 3 Minuten bei Raumtemperatur gerührt. Die gebildete Suspension wurde bei 0°C zu einer Lösung von 6,52 g (23,7 mmol) 2,4-Dichlor-5-jod-pyrimidin in 65 ml Acetonitril gegeben. Der Ansatz wurde 4 Stunden bei 40°C gerührt und anschließend mit verdünnter Natriumchlorid-Lösung versetzt. Man extrahierte mit Essigester (2x). Die vereinten organischen Phasen wurden getrocknet (Na₂SO₄), filtriert und eingeengt. Der erhaltene Rückstand wurde chromatographisch (Hexan / Essigester 4:1) gereinigt. Man erhielt 4,12 g (9,8 mmol; Ausbeute: 41%) des Produktes.
¹H-NMR (400 MHz, DMSO): δ = 8.78 (s, 1H), 7.29 (m, 5H), 5.27 (m, 1H), 4.64 (d, 1H), 4.53 (d, 1H), 3.73 (m, 1H), 1.30 (d, 3H), 1.19 (d, 3H).

### Verbindung 1.3

### 4-((1R,2R)-2-Benzyloxy-1-methyl-propoxy)-2-chlor-5-trifluormethyl-pyrimidin

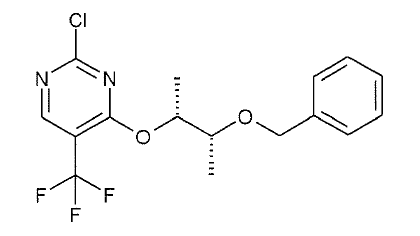

Eine Lösung von 4,66 g (11,1 mmol) 4-((1R,2R)-2-Benzyloxy-1-methyl-propoxy)-2-chlor-5-jod-pyrimidin in 15,8 ml NMP und 15,8 ml THF wurde bei Raumtemperatur unter Rühren mit 3,82 g (20,0 mmol) Kupfer(1)jodid, 0,97 g (16,7 mmol) Kaliumfluorid und 2,47 ml (16,7 mmol) (Trifluormethyl)-trimethylsilan versetzt. Der Ansatz wurde 5,5 Stunden bei 80°C gerührt. Nach dem Erkalten wurde der Ansatz in verdünnte Natriumchlorid-Lösung gegeben und mit Essigester extrahiert (2x). Die vereinten organischen Phasen wurden getrocknet (Na₂SO₄), filtriert und eingeengt. Der erhaltene Rückstand wurde chromatographisch (Hexan / Essigester 4: 1) gereinigt. Man erhielt 2,17 g (6,0 mmol; Ausbeute: 54%) des Produktes.
¹H-NMR (400 MHz, DMSO): δ = 8.81 (s, 1H), 7.21 (m, 5H), 5.40 (m, 1H), 4.57 (d, 1H), 4.42 (d, 1H), 3.70 (m, 1H), 1.28 (d, 3H), 1.13 (d, 3H).

### Verbindung 1.3 wurde alternativ auch durch folgende Vorschrift hergestellt:

Eine Lösung von 5,00 g (23,0 mmol) 2,4-Dichlor-5-trifluormethyl-pyrimidin und 5,40 g (30,0 mmol) (2R,3R)-3-Benzyloxy-butan-2-ol in 60 ml Diethylether und 65 ml Acetonitril wurde bei 0°C mit 1,21 g (27,7 mmol) Natriumhydrd (55%ig), aufgeteilt in 3 Portionen, versetzt und anschließend 90 Minuten bei 15°C gerührt. Der Ansatz wurde mit verdünnter Natriumchlorid-Lösung versetzt und mit Essigester extrahiert (3x). Die vereinten organischen Phasen wurden getrocknet (Na₂SO₄), filtriert und eingeengt. Der erhaltene Rückstand wurde chromatographisch (Hexan / Essigester 95:5) gereinigt. Man erhielt 1,60 g (4,4 mmol; Ausbeute: 19%) des Produktes.

### Verbindung 1.4

### [4-((1R,2R)-2-Benzyloxy-1-methyl-propoxy)-5-trifluormethyl-pyrimidin-2-yl]-(4-methylsulfanyl-phenyl)-amin

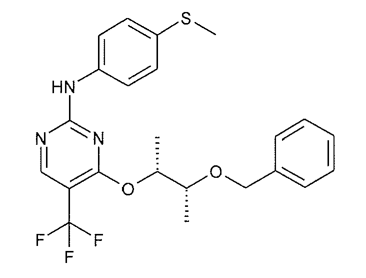

600 mg (1,66 mmol) 4-((1R,2R)-2-Benzyloxy-1-methyl-propoxy)-2-chlor-5-trifluormethyl-pyrimidin und 0,20 ml (1,66 mmol) 4-Methylsulfanyl-phenylamin in 8,10 ml Acetonitril wurden mit 0,42 ml einer 4N Lösung von Chlorwasserstoff in Dioxan versetzt und über Nacht bei 60°C gerührt. Nach dem Abkühlen wurde der Ansatz mit Essigester verdünnt und mit gesättigter Natriumhydrogencarbonat-Lösung gewaschen. Die organische Phase wurde getrocknet (Na₂SO₄), filtriert und eingeengt. Der erhaltene Rückstand wurde chromatographisch gereinigt (Hexan /Essigester 4: 1). Man erhielt 420 mg (0,91 mmol; Ausbeute: 54%) des Produktes.

¹H-NMR (400 MHz, DMSO): δ = 10.09 (s, 1H), 8.47 (s, 1H), 7.62 (m, 2H), 7.23 (m, 7H), 5.44 (m, 1H), 4.54 (d, 1H), 4.44 (d, 1H), 3.71 (m, 1H), 2.38 (s, 3H), 1.27 (d, 3H), 1.13 (d, 3H).

### Verbindung 1.5

### (2R,3R)-3-[2-(4-Methylsulfanyl-phenylamino)-5-trifluormethyl-pyrimidin-4-yloxy]-butan-2-ol

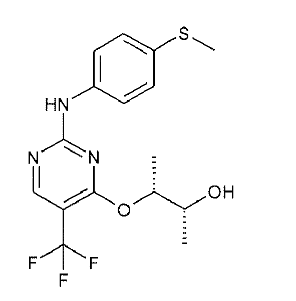

Eine Lösung von 200 mg (0,43 mmol) [4-((1 R,2R)-2-Benzyloxy-1-methyl-propoxy)-5-trifluormethyl-pyrimidin-2-yl]-(4-methylsulfanyl-phenyl)-amin in 20 ml Chloroform wurde unter Rühren bei 0°C tropfenweise mit 0,12 ml (0,86 mmol) Trimethylsilyljodid versetzt. Der Ansatz wurde auf Raumtemperatur erwärmt und über Nacht gerührt. Im Verlauf der nächsten 24 Stunden wurde der Ansatz noch mit 0,85 ml (6,12 mmol) Trimethylsilyljodid, aufgeteilt in 4 Portionen, versetzt. Der Ansatz wurde mit Methanol gequencht, mit gesättigter Natriumchlorid-Lösung versetzt und gegen Essigester extrahiert (3x). Die vereinten organischen Phasen wurden über einem Whatman-Filter filtriert und eingeengt. Der erhaltene Rückstand wurde chromatographisch (Hexan / Essigester 1:1) gereinigt. Man erhielt 70 mg (0,19 mmol; Ausbeute: 43%) des Produktes.
¹H-NMR (400 MHz, DMSO): δ = 10.06 (s, 1H), 8.41 (s, 1H), 7.63 (m, 2H), 7.21 (m, 2H), 5.23 (m, 1H), 4.82 (d, 1H), 3.80 (m, 1H), 2.41 (s, 3H), 1.22 (d, 3H), 1.06 (d, 3H).

### b) Herstellung des Endproduktes

Eine Lösung von 60 mg (0,16 mmol) (2R,3R)-3-[2-(4-Methylsulfanyl-phenylamino)-5-trifluormethyl-pyrimidin-4-yloxy]-butan-2-ol in 0,8 ml Acetonitril wurde mit 1 mg (0,005 mmol) Eisen(III)-chlorid und 40 mg (0,18 mmol) Perjodsäure versetzt und 3 Stunden bei Raumtemperatur gerührt. Der Ansatz wurde mit verdünnter Natriumthiosulfat-Lösung versetzt und mit DCM extrahiert (2x). Die vereinten organischen Phasen wurden über einem Whatman-Filter filtriert und eingeengt. Der erhaltene Rückstand wurde chromatographisch (DCM / EtOH 1:1) gereinigt. Man erhielt 53 mg (0,14 mmol; Ausbeute: 85%) des Produktes.
¹H-NMR (400 MHz, DMSO): δ = 10.35 (s, 1H), 8.52 (s, 1H), 7.89 (m, 2H), 7.62 (m, 2H), 5.26 (m, 1H), 4.86 (d, 1H), 3.81 (m, 1H), 2.69 (s, 3H), 1.25 (d, 3H), 1.07 (d, 3H). MS: 390 (ESI+)

### Beispiel 2

### (R)-3-[2-(4-Methansulfinyl-phenylamino)-5-trifluormethyl-pyrimidin-4-yloxy]-2-methyl-butan-2-ol

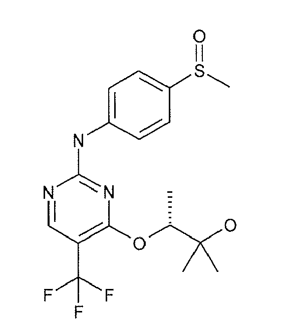

### 2a) Herstellung der Zwischenprodukte

### Verbindung 2.1.

### (R)-2-Methyl-butan-2,3-diol

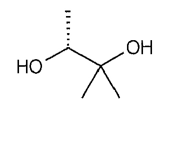

Eine Lösung von 10,0 g (96,1 mmol) (R)-(+)-Milchsäuremethylester in 20 ml THF wurde langsam zu 160 ml (480,0 mmol) einer eisgekühlten 3 N Lösung von Methylmagnesiumchlorid in THF getropft. Der Ansatz wurde zunächst langsam auf Raumtemperatur erwärmt und anschließend 30 Minuten refluxiert. Nach dem Abkühlen wurde der Ansatz auf eine gesättigte Ammoniumchlorid-Lösung gegeben und mit Essigester extrahiert (3x). Die vereinten organischen Phasen wurden über einem Whatman Filter filtriert und eingeengt. Man erhielt 4,5 g (43,1 mmol) des Rohproduktes, das ohne weitere Reinigung eingesetzt wurde.

¹H-NMR (400 MHz, DMSO): δ = 4.21 (d, 1H), 3.93 (s, 1H), 3.29 (m, 1H), 0.97 (m, 9H).

### Verbindung 2.2.

### (R)-3-(2-Chlor-5-jod-pyrimidin-4-yloxy)-2-methyl-butan-2-ol

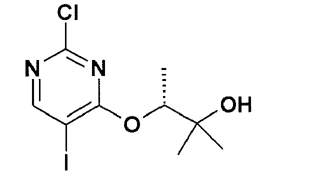

Eine Lösung von 4,40 g (42,3 mmol) (R)-2-Methyl-butan-2,3-diol in 83 ml Diethylether wurde unter Rühren bei 0°C portionsweise mit 1,84 g (42,3 mmol) Natriumhydrid (55%) versetzt und 10 Minuten gerührt. Man rührte weitere 3 Minuten bei Raumtemperatur und gab den Ansatz anschließend zu einer eisgekühlten Lösung von 9,68 g (35,2 mmol) 2,4-Dichlor-5-jod-pyrimidin in 97 ml Acetonitril. Der Ansatz wurde 4 Stunden bei 40°C gerührt und nach dem Erkalten mit Eis und gesättigter Natriumchlorid-Lösung versetzt. Man extrahierte mit Essigester (3x). Die vereinten organischen Phasen wurden getrocknet (Na₂SO₄), filtriert und eingeengt. Der erhaltene Rückstand wurde chromatographisch (Hexan / Essigester 4: 1) gereinigt. Man erhielt 4,96 g (14,5 mmol; Ausbeute: 41 %) des Produktes.

¹H-NMR (400 MHz, DMSO): δ = 8.73 (s, 1H), 4.96 (q, 1H), 4.62 (s, 1H), 1.21 (d, 3H), 1.13 (s, 6H). ES: 343 (CI+),

### Verbindung 2.3.

### 2-Chlor-4-[(R)-1,2-dimethyl-2-(tetrahydro-pyran-2-yloxy)-propoxy]-5-jod-pyrimidin

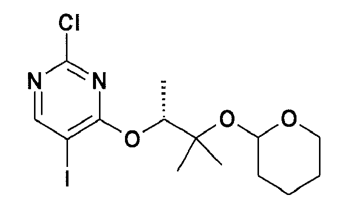

Eine Lösung von 4,96 g (14,5 mmol) (R)-3-(2-Chlor-5-jod-pyrimidin-4-yloxy)-2-methyl-butan-2-ol in 30 ml DCM wurde mit 2,64 ml (29,0 mmol) Dihydropyran und 0,36 g (1,5 mmol) Pyridiniumtosylat versetzt und 22 Stunden bei Raumtemperatur gerührt. Der Ansatz wurde mit DCM verdünnt und mit gesättigter Natriumhydrogencarbonat-Lösung gewaschen. Die organische Phase wurde getrocknet (Na₂SO₄), filtriert und eingeengt. Der erhaltene Rückstand wurde chromatographisch (Hexan / Essigester 4:1) gereinigt. Man erhielt 5,50 g (12,9 mmol; Ausbeute: 89%) des Diastereomerengemisches.

¹H-NMR (400 MHz, DMSO): δ = 8.75 (s, 1H), 8.74 (s, 1H), 5.15 (m, 2H), 4.91 (m, 2H), 3.70 (m, 2H), 3.30 (m, 2H), 1.31 (m, 30H).

### Verbindung 2.4.

### 2-Chlor-4-[(R)-1,2-dimethyl-2-(tetrahydro-pyran-2-yloxy)-propoxy]-5-trifluormethyl-pyrimidin

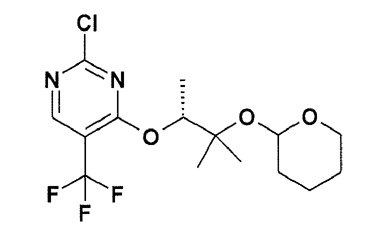

Eine Lösung von 1,00 g (2,34 mmol) 2-Chlor-4-[(R)-1,2-dimethyl-2-(tetrahydro-pyran-2-yloxy)-propoxy]-5-jod-pyrimidin in 3,3 ml NMP und 3,3 ml THF wurde bei Raumtemperatur mit 1,61 g (8,44 mmol) Kupfer(1)jodid, 0,41 g (7,03 mmol) Kaliumfluorid und 1,04 ml (7,03 mmol) (Trifluormethyl)-trimethylsilan versetzt. Der Ansatz wurde 2 Stunden bei 90°C gerührt. Nach dem Erkalten wurde der Ansatz in verdünnte Natriumchlorid-Lösung gegeben und mit Essigester extrahiert (3x). Die vereinten organischen Phasen wurden getrocknet (Na₂SO₄), filtriert und eingeengt. Der erhaltene Rückstand wurde chromatographisch (Hexan / Essigester 4:1) gereinigt. Man erhielt 0,53 g (1,43 mmol; Ausbeute: 61 %) des Produktes.

¹H-NMR (400 MHz, DMSO): δ = 8.84 (s, 1H), 5.32 (m, 1H), 4.85 (m, 1H), 3.68 (m, 1H), 3.30(m, 1H), 1.31 (m, 15H).

### b) Herstellung des Endproduktes

150 mg (0,41 mmol) 2-Chlor-4-[(R)-1,2-dimethyl-2-(tetrahydro-pyran-2-yloxy)-propoxy]-5-trifluormethyl-pyrimidin und 38 mg (0,24 mmol) 4-Methansulfinylphenylamin in 3,8 ml Ethanol wurden 3 Stunden bei 70°C gerührt. Der Ansatz wurde zur Trockene eingeengt und der Rückstand mittels HPLC gereinigt. Man erhielt 28 mg (0,07 mmol; Ausbeute: 29%) des Produktes.

| | | | |
|---|---|---|---|
| Säule: | XBridge C18 5µ 100x30 mm | | |
| Eluent A: | H₂O / 0.1% HCOOH | | |
| Eluent B: | Acetonitril | | |
| Gradient: | 0 min | 99%A | 1%B |
| | 1.00 min | 99%A | 1%B |
| | 7.50 min | 1%A | 99%B |
| | 10.00 min | 1%A | 99%B |
| Fluß: | 50,0 mL/min | | |
| Detektor: | DAD scan range 210-400 nm | | |
| | MS ESI+, ESI-, scan range 160-1000 m/z | | |
| Temperatur: | Raumtemperatur | | |

¹H-NMR (400 MHz, DMSO): δ = 10.36 (s, 1H), 8.53 (s, 1H), 7.90 (m, 2H), 7.61 (m, 2H), 5.12 (m, 1H), 4.66 (s, 1H), 2.69 (s, 3H), 1.27 (d, 3H), 1.12 (s, 6H). MS: 404 (ESI+)

### Beispiel 3

### Assay 1: CDK1/CycB Kinase Assay

Rekombinante CDK1- und CycB-GST-Fusionsproteine, gereinigt aus Bakulovirusinfizierten Insektenzellen (Sf9), wurden von ProQinase GmbH, Freiburg, gekauft. Das als Kinase-Substrat verwendet Histon IIIS ist über die Fa. Sigma käuflich zu erwerben.

CDK1/CycB (200 ng/Messpunkt) wurde für 10 min bei 22°C in Anwesenheit verschiedener Konzentrationen an Testsubstanzen (0 µM, sowie innerhalb des Bereiches 0,001 - 10 µM) in Assaypuffer [50 mM Tris/HCl pH8,0; 10 mM MgCl₂; 0,1 mM Na ortho-Vanadat; 1,0 mM Dithiothreitol; 0,5 µM Adenosintrisphosphat (ATP); 10 µg/Messpunkt Histon IIIS; 0,2 µCi/Messpunkt ³³P-gamma ATP; 0,05% NP40; 1,25% Dimethylsulfoxid] inkubiert. Die Reaktion wurde durch Zugabe von EDTA-Lösung (250 mM; pH8,0; 15 µl/Messpunkt) gestoppt.

Von jedem Reaktionsansatz wurden 15 µl auf P30 Filterstreifen (Fa. Wallac) aufgetragen, und nicht-eingebautes ³³P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex^{™} A, Fa. Wallac) bedeckt und für 1 Stunde bei 90°C eingebrannt. Die Menge an eingebautem ³³P (Substratphosphorylierung) wurde durch Szintillationsmessung in einem Gamma-Strahlungsmessgerät (Wallac) bestimmt. Die Messdaten wurden normalisiert auf 0% Inhibition (Enzymreaktion ohne Inhibitor) und 100% Inhibition (alle Assaykomponenten außer Enzym). Die Bestimmung der 1C50 Werte erfolgte mittels eines 4-Parameter Fits unter Benutzung firmeneigener Software.

### Assay 2: CDK2/CycE Kinase Assay

Rekombinante CDK2- und CycE-GST-Fusionsproteine, gereinigt aus Bakulovirusinfizierten Insektenzellen (Sf9), wurden von ProQinase GmbH, Freiburg, gekauft. Histon IIIS, das als Kinase-Substrat verwendet wurde, wurde bei der Fa. Sigma gekauft.

CDK2/CycE (50 ng/Messpunkt) wurde für 10 min bei 22°C in Anwesenheit verschiedener Konzentrationen an Testsubstanzen (0 µM, sowie innerhalb des Bereiches 0,001 - 10 µM) in Assaypuffer [50 mM Tris/HCl pH8,0; 10 mM MgCl₂; 0,1 mM Na ortho-Vanadat; 1,0 mM Dithiothreitol; 0,5 µM Adenosintrisphosphat (ATP); 10 µg/Messpunkt Histon IIIS; 0,2 µCi/Messpunkt ³³P-gamma ATP; 0,05% NP40; 1,25% Dimethylsulfoxid] inkubiert. Die Reaktion wurde durch Zugabe von EDTA-Lösung (250 mM; pH8,0; 15 µl/Messpunkt) gestoppt.

Von jedem Reaktionsansatz wurden 15 µl auf P30 Filterstreifen (Fa. Wallac) aufgetragen, und nicht-eingebautes ³³P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeItiLex^{™} A, Fa. Wallac) bedeckt und für 1 Stunde bei 90°C eingebrannt. Die Menge an eingebautem ³³P (Substratphosphorylierung) wurde durch Szintillationsmessung in einem Gamma-Strahlungsmessgerät (Wallac) bestimmt. Die Messdaten wurden normalisiert auf 0% Inhibition (Enzymreaktion ohne Inhibitor) und 100% Inhibition (alle Assaykomponenten außer Enzym). Die Bestimmung der 1C50 Werte erfolgte mittels eines 4-Parameter Fits unter Benutzung firmeneigener Software.

### Ergebnisse aus den Enzymassays

| | CDK1/CycB | CDK2/CycE |
|---|---|---|
| Bsp. | (Assay 1) | (Assay 2) |
| | Konzentration der halbmaximalen Inhibition der Enzymaktivität, IC50 | |
| | [nM] | |
| 1 | 7 | 5 |
| 2 | 30 | 12 |

### Schlußfolgerungen aus den Enzymassays

Die Beispielverbindungen stellen potente, nanomolare Inhibitoren der Zyklin-abhängigen Kinasen-1 und -2 dar.

## Patentansprüche

1. Verbindungen der allgemeinen Formel (I), 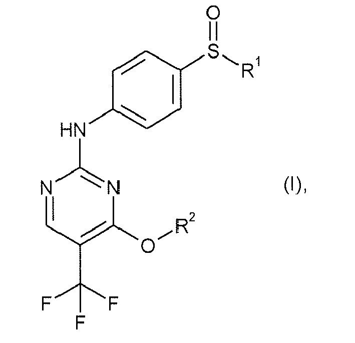 in der
R¹ für einen C₁-C₆-Alkyl-, C₂-C₆-Alkenyl-, C₂-C₆-Alkinylrest oder einen C₃-C₇-Cycloalkyl oder Phenylring steht, jeweils gegebenenfalls mit Hydroxy, -NR³R⁴, Cyano, Halogen, -CF₃, C₁-C₆-Alkoxy, -OCF₃ und/oder C₁-C₆-Alkyl ein- oder mehrfach, gleich oder verschieden substituiert, und
R² für einen C₁-C₁₀-Alkyl-, C₃-C₁₀-Alkenyl- oder C₃-C₁₀-Alkinylrest oder einen C₃-C₇-Cycloalkylring steht, jeweils gegebenenfalls ein- oder mehrfach, gleich oder verschieden substituiert mit
a) Halogen, Hydroxy, -NR³R⁴, Cyano, -CF₃, -OCF₃, und/oder
b) C₁-C₆-Alkoxy, C₁-C₆-Alkyl, C₂-C₆-Alkenyl, C₂-C₆-Alkinyl, oder C₃-C₈-Cycloalkyl, -O-CH₂-Phenyl, Cₙ-Alkoxycarbonyl, jeweils gegebenenfalls selbst ein- oder mehrfach, gleich oder verschieden mit Halogen, Hydroxy, einem C₁-C₆-Alkyl, C₁-C₆- Alkoxy, -NR³R⁴, -CF₃ und/oder -OCF₃ substituiert, und
R³ und R⁴ unabhängig voneinander für Wasserstoff und/oder einen C₁-C₆- Alkylrest, C₂-C₆-Alkenylrest, C₃-C₈-Cycloalkyl- und/oder Phenylring, einen Heterocyclylring mit 3 bis 8 Ringatomen und/oder einen monocyclischen Heteroarylring, gegebenenfalls mit Hydroxy,
R³ und R⁴ -NR⁵R⁶, Cyano, Halogen, -CF₃, C₁-C₆-Alkoxy und/oder -OCF₃ ein- oder mehrfach, gleich oder verschieden substituiert sind, oder gemeinsam mit dem Stickstoffatom einen 5- bis 7-gliedrigen Ring bilden, der gegebenenfalls zusätzlich zum Stickstoffatom 1 oder 2 weitere Heteroatome enthält und der mit Hydroxy, -NR⁵R⁶, Cyano, Halogen, -CF₃, C₁-C₆-Alkoxy und/oder -OCF₃ ein- oder mehrfach, gleich oder verschieden substituiert sein kann, und
R⁵ und R⁶ unabhängig voneinander für Wasserstoff oder einen C₁-C₆-Alkylrest stehen, der gegebenenfalls mit Hydroxy, Cyano, Halogen, -CF₃, C₁-C₆-Alkoxy und/oder -OCF₃ ein- oder mehrfach, gleich oder verschieden substituiert ist.
sowie deren Salze, Diatereomere und Enantiomere.

2. Verbindungen gemäß Anspruch 1,
wobei
R¹ für einen C₁-C₆-Alkyl-, C₂-C₆-Alkenyl-, C₂-C₆-Alkinylrest oder einen C₃-C₇-Cycloalkyl oder Phenylring steht, jeweils gegebenenfalls mit Hydroxy, Cyano, Halogen, -CF₃, C₁-C₆-Alkoxy, -OCF₃ und/oder C₁-C₆-Alkyl ein- oder mehrfach, gleich oder verschieden substituiert,
sowie deren Salze, Diatereomere und Enantiomere.

3. Verbindungen gemäß einem der Ansprüche 1 oder 2,
wobei
R² für einen C₁-C₁₀-Alkyl-, C₃-C₁₀-Alkenyl- oder C₃-C₁₀-Alkinylrest oder einen C₃-C₇-Cycloalkylring steht, jeweils gegebenenfalls ein- oder mehrfach, gleich oder verschieden substituiert mit Halogen, Hydroxy, Cyano, -CF₃, -OCF₃, und/oder C₁-C₆-Alkoxy, C₁-C₆-Alkyl, jeweils gegebenenfalls selbst ein- oder mehrfach, gleich oder verschieden mit Halogen oder Hydroxy,
sowie deren Salze, Diatereomere und Enantiomere.

4. Verbindungen gemäß einem der Ansprüche 1 bis 3,
wobei
R¹ für einen C₁-C₆-Alkyl- oder C₂-C₆-Alkenylrest oder einen C₃-C₇- Cycloalkyl- oder Phenylring steht, jeweils gegebenenfalls mit Hydroxy, Cyano, Halogen und/oder C₁-C₆-Alkyl ein- oder mehrfach, gleich oder verschieden substituiert,
sowie deren Salze, Diatereomere und Enantiomere.

5. Verbindungen gemäß einem der Ansprüche 1 bis 4,
wobei
R² für einen C₂-C₆-Alkyl-, C₃-C₆-Alkenyl- oder C₃-C₆-Alkinylrest oder einen C₃-C₇-Cycloalkylring steht, der gegebenenfalls ein- oder mehrfach substituiert ist mit Hydroxy, Halogen, -CF₃ und/oder C₁-C₃- Alkoxy,
sowie deren Salze, Diatereomere und Enantiomere.

6. Verbindungen gemäß einem der Ansprüche 1 bis 5,
wobei
R² für die Gruppe mit der Teilformel (I-_{R}²) steht, 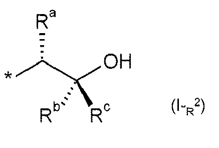
in der
R^{a} für eine Methyl-, Ethyl-, Propyl- oder Isopropylgruppe steht, und
R^{b} und R^{c} unabhängig voneinander für Wasserstoff, eine Methyl- oder Ethylgruppe stehen.
sowie deren Salze, Diatereomere und Enantiomere.

7. Verbindungen gemäß Anspruch 6,
wobei
R^{a} und R^{b} für eine Methylgruppe und R^{c} für Wasserstoff oder eine Methylgruppe stehen,
sowie deren Salze, Diatereomere und Enantiomere.

8. Verbindungen der allgemeinen Formel (la) 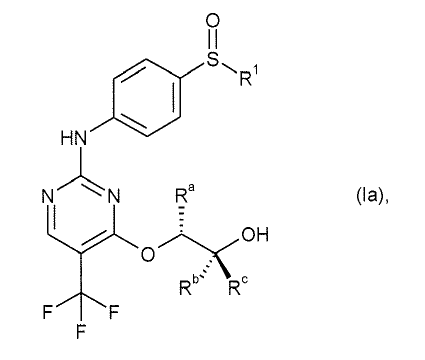 in der
R¹ für eine Methylgruppe steht, und
R^{a} und R^{b} für eine Methylgruppe stehen, und
R^{c} für Wasserstoff oder eine Methylgruppe steht,
sowie deren Salze, Diatereomere und Enantiomere.

9. Verfahren zur Herstellung der Verbindungen gemäß einem der Ansprüche 1 bis 8 umfassend mindestens einer der Schritte
a₁) Funktionalisierung der 4-Position von 2,4-Dichlor-5-jod-pyrimidin **(1)** durch Umsetzung mit einem Alkohol der Formel **(2)** unter Bildung eines Intermediates der Formel **(3),** 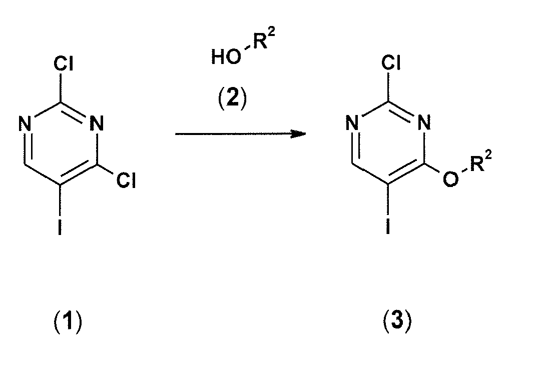 und nachfolgende Umsetzung von Intermediat **(3)** unter Bildung des 5-CF₃ Intermediates **(4)** 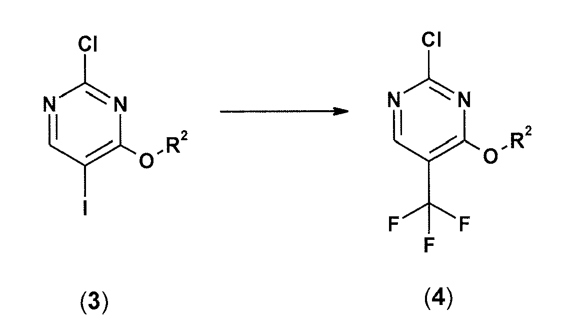 oder alternativ
a₂) direkte Umsetzung von 2,4-Dichlor-5-trifluormethyl-pyrimidin **(5)** und einem Alkohol der Formel **(2)** unter Bildung des 5-CF₃ Intermediates **(4).** 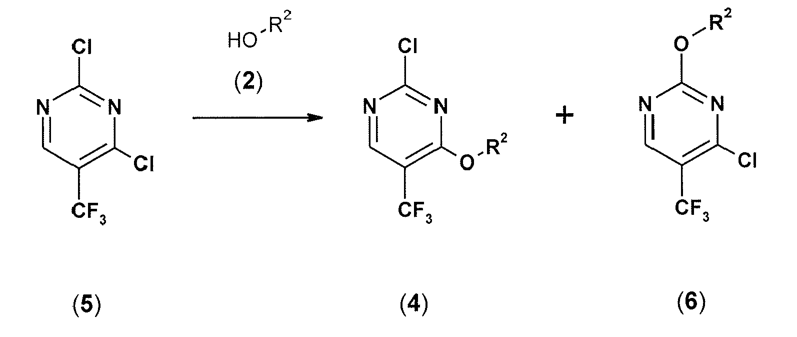
b₁) Oxidation eines Thioethers der Formel **(7)** zum Sulfoxid der Formel **(8)** 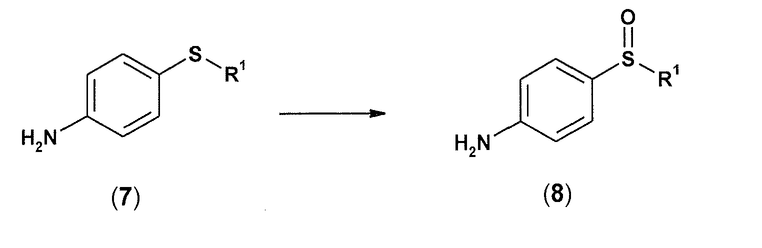 und anschließende Kupplung der Verbindungen der Formel **(4)** und **(8),** 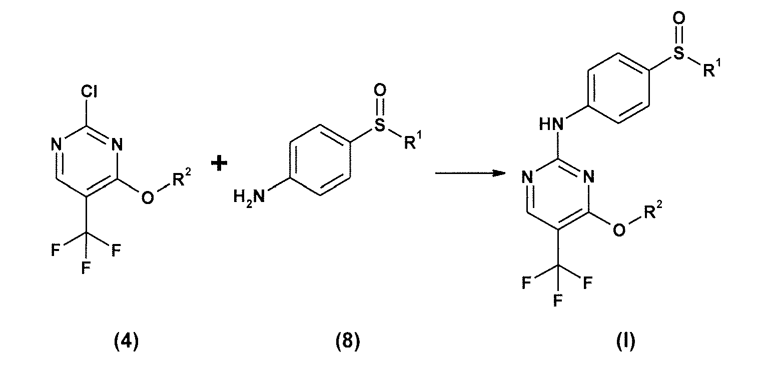 oder alternativ
b₂) Kupplung der Intermediate **(4)** und **(7)** unter Bildung des Produktes **(9):** 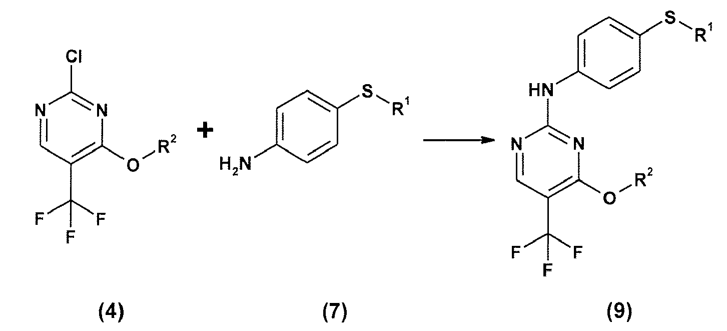 und anschließend Oxidation von **(9)** zum Sulfoxid, 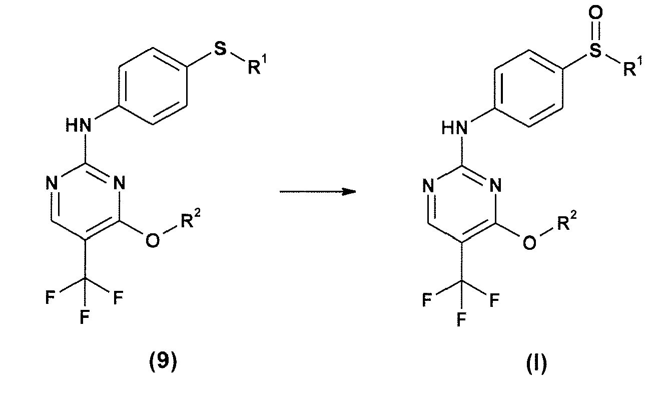 wobei die Substituenten R¹ und R² die in der allgemeinen Formel (1) gemäß einem der Ansprüche 1 bis 7 angegebenen Bedeutungen aufweisen.

10. Verbindungen gemäß einem der Ansprüche 1 bis 8 zur Verwendung als Arzneimittel.

11. Verwendung einer Verbindung gemäß einem der Ansprüche 1 bis 8 zur Herstellung eines Arzneimittels zur Behandlung von Krebs.

12. Verbindungen gemäß einem der Ansprüche 1 bis 8 zur Verwendung als Arzneimittel gegen Krebs.

13. Pharmazeutische Formulierung enthaltend eine Verbindung gemäß einem der Ansprüche 1 bis 8.
